(19) Europäisches Patentamt
European Patent Office
Office européen des brevets

(11) Publication number : **0 496 692 A1**

(12) # EUROPEAN PATENT APPLICATION

(21) Application number : **92500008.5**

(22) Date of filing : **23.01.92**

(51) Int. Cl.[5] : **C07D 295/12,** C07D 207/40, C07D 333/38, C07D 333/28, C07D 333/24, C07D 333/20, C07D 295/22, C07D 295/18, C07D 213/82, C07D 207/34

(30) Priority : **24.01.91 ES 9100182**

(43) Date of publication of application : **29.07.92 Bulletin 92/31**

(84) Designated Contracting States : **AT BE CH DE DK FR GB GR IT LI LU NL SE**

(71) Applicant : **FABRICA ESPANOLA DE PRODUCTOS QUIMICOS Y FARMACEUTICOS, S.A. (FAES)**
**c/ Maximo Aguirre, 14**
**E-48940 Leioa-Lamiaco (Vizcaya) (ES)**

(72) Inventor : **Orjales Venero,Aurelio**
**Po del Puerto, 24**
**Neguri (Vizcaya) (ES)**
Inventor : **Alonso Cirés, Luisa**
**Plaza Xaho, 2 - 5oC**
**E-48940 Leioa (Vizcaya) (ES)**

(74) Representative : **Isern-Cuyas, Maria Luisa**
**Paseo de la Castellana 131, Bajo C**
**E-28046 Madrid (ES)**

(54) **2-Methoxyphenylpiperazine derivatives.**

(57) New 2-methoxyphenylpiperazine derivatives are described with the following general formula :

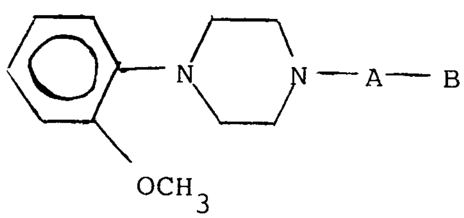

where A is a carbonyl group, an alkylaminocarbonyl or alkylaminomethylene group, and B is a substituted aryl, heteroaryl substituted or otherwise, arylalkyl, heteroarylalkyl or cycloalkyl group, and their pharmaceutically acceptable addition salts. These compounds have a pharmacologic activity at the level of serotonin 5-$HT_{1A}$ receptors.

EP 0 496 692 A1

The present invention relates to a number of new 2-methoxyphenylpiperazine derivatives, with formula I, and their addition salts with pharmaceutically acceptable acids.

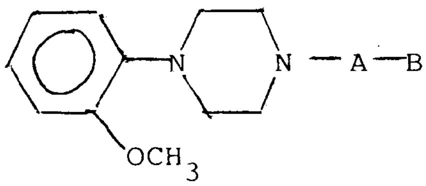

I

In formula I, A is a -CO- group, a $-(CH_2)_n$ HNCO- group, with n values lying between two and four, or a $-(CH_2)_nHNCH_2-$ group, with n values lying between two and four, and B is an aryl, heteroaryl, arylalkyl, heteroarylalkyl or cycloalkyl group, which may be substituted or otherwise by a halogen, preferably chlorine, and/or by a methoxy, methyl or amino group. Among the pharmaceutically acceptable acids for preparing addition salts are hydrochloric, fumaric, oxalic and like acids.

The 2-methoxyphenylpiperazine derivatives subject of this patent are obtained by reacting an amine with formula

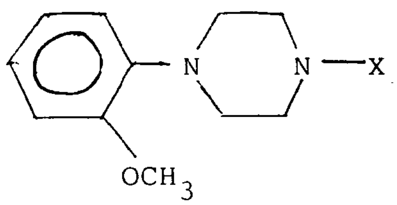

II

where X can be hydrogen or an alkylenamino group, wherein the carbonated chain has two, three or four carbon atoms, with benzoyl chloride substituted in one or several positions by a halogen, an amino group or a methoxy group; or with a heteroaromatic acid chloride, wherein the heteroatom can be oxygen, sulphur or nitrogen, forming part of a cycle of five or six links and which may be substituted by one or more methyl groups, a halogen which can be chlorine, or an aromatic ring; or with a cyclo- or bicycloalkanoic or -alkenoic acid. The reaction takes place in the presence of a suitable organic solvent, such as dichloromethane, tetrahydrofurane or ether, at room temperature, in a basic medium by adding triethylamine, pyridine, sodium bicarbonate or other organic and inorganic bases.

The compounds wherein A is an alkylaminomethylene group are obtained by reducing the relevant alkylaminocarbonyl derivatives with a suitable reducing agent, such as sodium borohydride, sodium, aluminium and lithium hydride.

A preliminary survey determined the affinity of the compounds herein described by the $5\text{-}HT_{1A}$ receptors, using the technique initially set forth by Gozkan et al. (Nature, 305, 140-2, 1983) to bind and shift radioligands provided on tissue obtained from the front cortex of rats and using $[^3H]$-8-OH-DPAT as ligand. The compounds analysed show a degree of affinity by such receptors similar to the buspyrone molecule.

The following examples provide further details on the invention, without limiting it at all to the same.

Example 1

Preparing N-[2-[4-(2-methoxyphenyl)piperazin-1-yl]ethyl]adamantancarboxamide.

1.0 g of 1-(2-aminoethyl)-4-(2-methoxyphenyl)piperazine was dissolved in 20 ml of tetrahydrofurane, adding 2.1 ml of triethylamine. 1.0 g of adamantancarbonyl chloride dissolved in 20 ml of tetrahydrofurane was added dropwise upon the solution at 0°C. When the addition was completed, the solution was stirred for one hour at room temperature, whereupon 40 ml of water were added and extraction took place with 20 ml of dichloromethane, three times. The organic layer was separated and dried with anhydrous sodium sulphate. After distilling the solvent in a vacuum, 1.6 g of a white solid were obtained and purified by column chromatography, using dichloromethane/methanol (9/1) as eluent. M.P.: 112-4°C.

Example 2

Preparing N-[4-[4-(2-methoxyphenyl)piperazin-1-yl]butyl]-2-pyrrolcarboxamide.

1 g of 1-(4-aminobutyl)-4-(2-methoxyphenyl)piperazine was dissolved in 20 ml of dichloromethane, adding 1.5 g of pyridine. 1.0 g of pyrrocarbonyl chloride dissolved in 20 ml of dichloromethane was added dropwise at 0°C. The solution was stirred for one hour at 20°C whereupon 40 ml of water were added and extraction took place adding a further 20 ml of dichloromethane. The organic layer was dried with anhydrous sodium sulphate and the solvent was eliminated by distillation in a vacuum. 1.4 g of a solid were obtained, purified by column chromatography using dichloromethane/methanol (9/1) as eluent. M.P.: 173-4°C.

The novel and own findings submitted for a patent of invention to be granted are as contained in the following claims:

## Claims

**1.** New 2-methoxyphenylpiperazine derivatives, with the following general formula

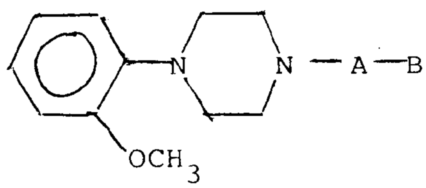

I

wherein A is a -CO- group, a $-(CH_2)_n$ HNCO- group, with n values lying between two and four, and B is an aryl, heteroaryl, arylalkyl, hetereoarylalkyl, cycloalkyl or bicycloalkyl group, substituted or otherwise by a halogen, preferably chlorine, and/or by a methoxy, methyl and amino group, and their addition salts with pharmaceutically acceptable acids.

**2.** New 2-methoxyphenylpiperazine derivatives, as in claim 1, characterised in being:

. N-[2-[4-(2-methoxyphenylpiperazin-1-yl]ethyl]-4-amino-5-chloro-2-methoxybenzamide.
. 1-(2-methoxyphenyl)-4-(2-thiophencarbonyl)piperazine.
. N-[2-[4-(2-methoxyphenyl)piperazin-1-yl]ethyl]-3-thiophencarboxamide.
. N-[2-[4-(2-methoxyphenyl)piperazin-1-yl]ethyl]phenylacetamide.
. N-[2-[4-(2-methoxyphenyl)piperazin-1-yl]ethyl]-2.4-dichlorobenzamide.
. 1-(2-methoxyphenyl)-4-(3-thiophencarbonyl)piperazine.
. 1-(5-methyl-2-thiophencarbonyl)-4-(2-methoxyphenyl)piperazine.
. N-[2-[4-(2-methoxyphenyl)piperazin-1-yl]ethyl]-5-methyl-2-thiophencarboxamide.
. 1-(3-chloro-2-thiophencarbonyl)-4-(2-methoxyphenyl)piperazine.
. 1-(3-methyl-2-thiophencarbonyl)-4-(2-methoxyphenyl)piperazine.
. 1-benzoyl-4-(2-methoxyphenyl)piperazine.
. 1-(2.4-dichlorobenzoyl)-4-(2-methoxyphenyl)piperazine.
. N-[2-[4-(2-methoxyphenyl)piperazin-1-yl]ethyl]-3-methyl-2-thiophencarboxamide.
. N-[2-[4-(2-methoxyphenyl)piperazin-1-yl]ethyl]-2-pyrrolcarboxamide.
. N-[2-[4-(2-methoxyphenyl)piperazin-1-yl]ethyl]-3-indolcarboxamide.
. N-[2-[4-(2-methoxyphenyl)piperazin-1-yl]ethyl]-3-thiophenacetamide.
. N-[3-[4-(2-methoxyphenyl)piperazin-1-yl]propyl]-3-indolcarboxamide.
. N-[4-[4-(2-methoxyphenyl)piperazin-1-yl]butyl]-3-pyridincarboxamide.
. N-[3-[4-(2-methoxyphenyl)piperazin-1-yl]propyl]-2-pyrrolcarboxamide.
. N-[4-[4-(2-methoxyphenyl)piperazin-1-yl]butyl]-2-pyrrolcarboxamide.
. N-[3-[4-(2-methoxyphenyl)piperazin-1-yl]propyl]-3-pyridincarboxamide.
. 1-(3-indolcarbonyl)-4-(2-methoxyphenyl)piperazine.
. N-[4-[4-(2-methoxyphenyl)piperazin-1-yl]butyl]-3-indolcarboxamide.
. N-[4-[4-(2-methoxyphenyl)piperazin-1-yl]butyl]-2.4-dichlorobenzamide.
. 1-(3-indolmethyl)-4-(2-methoxyphenyl)piperazine.
. N-[4-[4-(2-methoxyphenyl)piperazin-1-yl]butyl]-2-thiophencarboxamide.
. N-[4-[4-(2-methoxyphenyl)piperazin-1-yl]butyl]-3-methyl-2-thiophencarboxamide.

. N-[4-[4-(2-methoxyphenyl)piperazin-1-yl]butyl]-3-chloro-2-thiophencarboxamide.
. N-[4-[4-(2-methoxyphenyl)piperazin-1-yl]butyl]-3-methoxy-2-thiophencarboxamide.
. N-[4-[4-(2-methoxyphenyl)piperazin-1-yl]butyl]phenylacetamide.
. N-[4-[4-(2-methoxyphenyl)piperazin-1-yl]butyl]-2-furancarboxamide.
. N-[4-[4-(2-methoxyphenyl)piperazin-1-yl]butyl]-3-thiophenacetamide.
. N-[4-[4-(2-methoxyphenyl)piperazin-1-yl]butyl]-5-methyl-2-thiophencarboxamide.
. N-[4-[4-(2-methoxyphenyl)piperazin-1-yl]butyl]-3-thiophencarboxamide.
. N-[2-[4-(2-methoxyphenyl)piperazin-1-yl]ethyl]-5-benzimidazolcarboxamide.
. 1-(2-methoxyphenyl)-4-[2-(2-pyrrolmethylamino)ethyl]piperazine.
. 1-(2-methoxyphenyl)-4-[2-(3-methyl-2-thiophenmethylamino)ethyl]piperazine.
. N-[2-[4-(2-methoxyphenyl)piperazin-1-yl]-1-methyl-2-pyrrolcarboxamide.
. N-[2-[4-(2-methoxyphenyl)piperazin-1-yl]-ethyl-cys-bicyclo [3.3.0] octan-2-carboxamide.
. N-[4-[4-(2-methoxyphenyl)piperazin-1-yl]butyl-2-indolcarboxamide.
. N-[4-[4-(2-methoxyphenyl)piperazin-1-yl]butyl]-2-norbornancarboxamide.
. N-[4-[4-(2-methoxyphenyl)piperazin-1-yl]butyl]-5-norbornan-2-carboxamide.
. N-[2-[4-[4-(2-methoxyphenyl)piperazin-1-yl]ethyl]cyclopentancarboxamide.
. N-[4-[4-(2-methoxyphenyl)piperazin-1-yl]butyl]cyclopentancarboxamide.
. N-[4-[4-(2-methoxyphenyl)piperazin-1-yl]butyl]adamantancarboxamide.
. N-[2-[4-(2-methoxyphenyl)piperazin]-1-yl]ethyl]-2-norbornancarboxamide.
. N-[2-[4-(2-methoxyphenyl)piperazin-1-yl]ethyl]-5-norbornan-2-carboxamide.

**Claims for the following Contracting State: GR.**

**1.-** A PROCESS FOR OBTAINING NEW 2-METHOXYPHENYLPIPERAZINE DERIVATIVES, with the following general formula

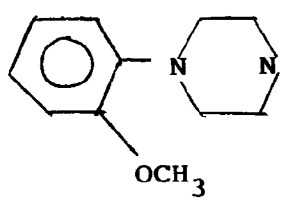

I

wherein A is a -CO- group, a $-(CH_2)_n$ HNCO- group, with n values lying between two and four, or a $-(CH_2)_nHNCH_2-$, with n values lying between two and four, and B is an aryl, heteroaryl, arylalkyl, heteroarylalkyl, cycloalkyl or bicycloalkyl group, substituted or otherwise by a halogen, preferably chlorine, and/or by a methoxy, methyl and amino group, and their addition salts with pharmaceutically acceptable acids, characterised in reacting an N-substituted piperazine with an acid chloride in a suitable solvent, such as dichloromethane, tetrahydrofurane or ether, in the presence of a base such as triethylamine, pyridine or sodium bicarbonate, for an hour, at room temperature.

**2.-** A PROCESS FOR OBTAINING NEW 2-METHOXYPHENYLPIPERAZINE DERIVATIVES, as in the above claim, characterised in that the N-substituted piperazine has the following formula

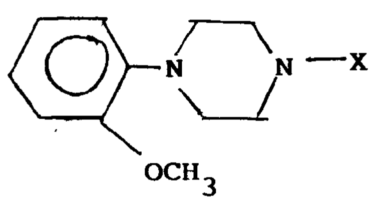

II

8

where X can be hydrogen or a $-(CH_2)_nNH_2-$ group, with n values lying between two and four.

**3.-** A PROCESS FOR OBTAINING NEW 2-METHOXYPHENYLPIPERAZINE DERIVATIVES, as in the previous claims, characterised in that the acid chloride is a benzoic acid chloride substituted in one or several positions by a halogen, preferably chlorine, an amino group or a methoxy group, or a heteroaromatic acid, wherein

the heteroatom can be oxygen, sulphur or nitrogen, forming part of cycles with five or six links, substituted or otherwise by one or more methyl halogen or phenyl groups, or an alkanoic or alkenoic acid, substituted by a heteroaromatic cycle or a phneyl group, or a cyclo- or bicycloarboxylic acid.

**4.-** A PROCESS FOR OBTAINING NEW 2-METHOXYPHENYLPIPERAZINE DERIVATIVES, as in the previous claims, characterised in that the compounds, wherein A is a $-(CH^2)_nHNCH_2$-group in the general formula I, are obtained by reduction with a suitable reducing agent, such as aluminium or lithium hydride, from the compounds wherein A is a $-(CH_2)_nHNCO-$ group in general formula I.

European Patent Office

# PARTIAL EUROPEAN SEARCH REPORT

which under Rule 45 of the European Patent Convention shall be considered, for the purposes of subsequent proceedings, as the European search report

Application Number

EP 92 50 0008

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 343 961 (AMERICAN HOME PRODUCTS CORP.) * Abstract; page 2, lines 1-53; page 5, scheme 1; claims * --- | 1,3-5 | C 07 D 295/12<br>C 07 D 207/40<br>C 07 D 333/38<br>C 07 D 333/28<br>C 07 D 333/24<br>C 07 D 333/20<br>C 07 D 295/22<br>C 07 D 295/18<br>C 07 D 213/82<br>C 07 D 207/34 |
| X | EP-A-0 186 796 (KYORIN PHARMACEUTICAL CO., LTD) * The whole document * --- | 1 | |
| X | EP-A-0 104 614 (CHUGAI SEIYAKU K.K.) * Page 1, line 1 - page 2, line 22; claims 1-3 * --- | 1,3 | |
| X | US-A-3 466 287 (SYDNEY ARCHER) * The whole document * & NL-A-6 713 659 (STERLING DRUG INC.) * Table 5, examples CCXCV, CCCXXII, CCCXLIII * --- | 1 | |
| A | EP-A-0 385 043 (FABRICA ESPANOLA DE PRODUCTOS QUIMICOS Y FARMACEUTICOS, S.A. (FAES)) * Abstract; claim 1 * --- -/- | 1 | TECHNICAL FIELDS SEARCHED (Int. Cl.5)<br>C 07 D |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely : 1-6

Claims not searched :

Reason for the limitation of the search:

See sheet -C-

| Place of search | Date of completion of the search | Examiner |
|---|---|---|
| THE HAGUE | 13-04-1992 | PAISDOR B. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)

Page 2

European Patent Office

# PARTIAL EUROPEAN SEARCH REPORT

Application Number
EP 92 50 0008

| DOCUMENTS CONSIDERED TO BE RELEVANT | | | CLASSIFICATION OF THE APPLICATION (Int. Cl.5) |
|---|---|---|---|
| Category | Citation of document with indication, where appropriate, of relevant passages | Relevant to claim | |
| P,A | EP-A-0 434 561 (ADIR ET CO.)<br>* Abstract; claims 1,6,9 *<br>----- | 1,3 | |
| | | | TECHNICAL FIELDS SEARCHED (Int. Cl.5) |

EPO FORM 1503 03.82 (P0410)

EP 92 50 0008 -C-

As the drafing of the claims is not clear and concise (Art. 83-84 EPC) and encompasses such an enormous amount of products, a complete search is not possible on economic grounds (see Guidelines for Examination in the EPO, Part B, Chapter III, 2). So the search has been limited (Rule 45) to the inventive concept as illustrated in the examples.